# EUROPÄISCHE PATENTSCHRIFT

(11) **EP 3 724 136 B1**
(45) Veröffentlichungstag und Bekanntmachung des Hinweises auf die Patenterteilung: **10.11.2021**
(21) Anmeldenummer: 18769173.8
(22) Anmeldetag: 12.09.2018
(51) Int. Cl.: C02F 9/00, C02F 1/26, C02F 103/26, C02F 1/44, C02F 1/04

(54) **VERFAHREN ZUR AUFBEREITUNG VON BEI DER HERSTELLUNG VON MODIFIZIERTEN STÄRKEN ANFALLENDEM ABWASSER**
PROCESS FOR THE TREATMENT OF WASTEWATER FORMED DURING THE PRODUCTION OF MODIFIED STARCHES
PROCÉDÉ DE TRAITEMENT D'EAU RÉSIDUAIRE PROVENANT DE LA PRODUCTION D'AMIDONS MODIFIÉS

(30) Priorität: 12.12.2017 AT 510282017
(43) Veröffentlichungstag der Anmeldung: 21.10.2020
(73) Patentinhaber: Gaisch, Franz, 4100 Ottensheim (AT)
(72) Erfinder: Gaisch, Franz, 4100 Ottensheim (AT)
(74) Vertreter: KLIMENT & HENHAPEL
(86) Internationale Anmeldenummer: PCT/EP2018/074578
(87) Internationale Veröffentlichungsnummer: WO 2019/115032

(56) Entgegenhaltungen:
- EP-A1- 2 796 418
- DE-A1- 19 800 335
- US-B1- 7 306 739

## Beschreibung

Die Erfindung betrifft ein Verfahren zur Aufbereitung von bei der Herstellung von Stärken, insbesondere von chemisch modifizierten Stärken anfallendem und gelöste Salze und organische Verbindungen enthaltendem Abwasser, gemäß dem Anspruch 1.

Modifizierte Stärken finden vielfältige Anwendungen in der Industrie, wobei Hauptanwendungsgebiete in der Lebensmittel- und Papierindustrie liegen. Sie können physikalisch, chemisch oder enzymatisch hergestellt werden. Bei chemisch modifizierten Stärken werden durch den Einsatz von Chemikalien Eigenschaften der Stärke an die jeweils geforderten Anforderungen angepasst. Damit ist es beispielsweise möglich die physikalischen und rheologischen Eigenschaften, sowie die Quellungs- und Wasserbindungseigenschaften der Stärke zu verändern. Die Stärke kann unter anderem aus den Rohstoffen Mais, Weizen, Kartoffel, Tapioka oder Reis gewonnen werden. Die chemische Modifizierung wird in wässriger Lösung bzw. Suspension durchgeführt, indem der in Wasser suspendierten Stärke ("Slurry") Chemikalien zugesetzt werden. Das erfindungsgemäß zu reinigende Abwasser entsteht bei der Abtrennung und der Auswaschung der modifizierten Stärke aus dem Reaktionsgemisch. Die Waschung erfolgt meist durch eine Hydrowaschzyklonanlage und die Abtrennung der Stärke meist durch Zentrifugalabscheider wie etwa Separatoren und/oder Zentrifugen.

Das nach Abtrennung und Auswaschung der modifizierten Stärke verbleibende Abwasser enthält hohe Konzentrationen an organischen Verbindungen und gelösten Salzen. Bei der Herstellung von Hydroxypropylstärke (HPS) beispielsweise fällt Abwasser mit hohen Konzentrationen von 1,2-Propandiol und Natriumsulfat an. In herkömmlicher Weise werden diese Abwässer in biologischen Kläranlagen gereinigt, wobei die organischen Verbindungen durch aerobe Prozesse abgebaut und in Biomasse ("sludge") umgewandelt werden. Der hohe Anteil an gelösten Salzen verursacht jedoch große Schwierigkeiten bei dieser biologischen Abwasserreinigung. Mit steigender Salzkonzentration wird die Biozönose in der biologischen Abwasserbehandlung gehemmt, dies kann zu Funktionsstörungen bis hin zur völligen Einstellung der biologischen Abwasserreinigung führen. Die hohe Sulfatkonzentration im Abwasser führt zusätzlich zu Korrosion in der Anlagentechnik und in den Betonbecken der Abwasserreinigungsanlagen. Die Salzfracht wird in der biologischen Abwasserbehandlungsanlage nicht abgebaut und gelangt daher über den Ablauf in den Vorfluter und belastet diesen. Zudem muss die in der biologischen Abwasserreinigungsstufe anfallende Biomasse ("Klärschlamm") zumeist kostenaufwändig entsorgt werden.

Weitere Verfahren zur Behandlung von Abwasser aus der Herstellung modifizierter Stärken wurden in der EP 2796418 A1 und der WO 2010/037156 A1 beschrieben. Verfahren zur Behandlung von Abwasser aus der Kartoffelverarbeitung wurden in der US 7,306,739 B1 und der DE 198 00 335 A1 beschrieben.

Es besteht somit das Ziel der Erfindung darin, die Behandlung des bei der Herstellung von Stärken, insbesondere von chemisch modifizierten Stärken anfallenden Abwassers zu verbessern und insbesondere ein Verfahren zur Aufbereitung des Abwassers unter Rückgewinnung verwertbarer Inhaltsstoffe bereitzustellen.

Diese Ziele werden durch die Merkmale von Anspruch 1 erreicht. Anspruch 1 bezieht sich auf ein Verfahren zur Aufbereitung von bei der Herstellung von Stärken, insbesondere von chemisch modifizierten Stärken anfallendem und gelöste Salze und organische Verbindungen enthaltendem Abwasser, bei dem erfindungsgemäß vorgeschlagen wird, dass das Abwasser oder ein die gelösten Salze und die organischen Verbindungen des Abwassers im Wesentlichen enthaltendes, vorbehandeltes Abwasser einem Membrantrennverfahren unterzogen wird, in der eine Trennung des dem Membrantrennverfahren zugeführten Abwassers in einen ersten Volumenstrom mit im Vergleich zum zugeführten Abwasser höherer Konzentration an gelösten Salzen und in einen zweiten Volumenstrom mit im Vergleich zum zugeführten Abwasser verringerter Konzentration an gelösten Salzen erfolgt, wobei der erste Volumenstrom einer thermischen Behandlung zur Abtrennung der gelösten Salze und eines dritten, einen Anteil der organischen Verbindungen des Abwassers enthaltenden Volumenstroms unterzogen wird, und der dritte Volumenstrom oder ein von ihm abgeleiteter Volumenstrom einer selektiven Auftrennung zur Abtrennung und Rückgewinnung von 1,2-Propandiol oder Ethylenglykol unterzogen wird. Erfindungsgemäß wird somit eine Trennung des Abwassers in unterschiedliche Volumenströme vollzogen, wobei die gelösten Salze in einem ersten Volumenstrom konzentriert werden, und zumindest ein Anteil der organischen Verbindungen in einem dritten Volumenstrom. Zumindest der im dritten Volumenstrom enthaltene Anteil der organischen Verbindungen steht in weiterer Folge einer Rückgewinnung zur Verfügung, wie noch näher ausgeführt werden wird. Die gelösten Salze werden durch thermische Behandlung des ersten Volumenstroms rückgewonnen, wobei die Rückgewinnung mittels thermischer Behandlung deshalb wirtschaftlich möglich ist, weil der erste Volumenstrom ein deutlich kleineres Volumen als das ursprünglich dem Membrantrennverfahren zugeleitete Abwasser aufweist. Tatsächlich erweist sich das erfindungsgemäße Verfahren alleine durch Rückgewinnung und Wiederverwertung der rückgewonnenen Salze trotz des Energieaufwands für die thermische Behandlung als wirtschaftlich. Werden zudem auch die im dritten Volumenstrom enthaltenen organischen Verbindungen 1,2-Propandiol oder Ethylenglykol rückgewonnen und wiederverwertet, gestaltet sich die wirtschaftliche Bilanz des erfindungsgemäßen Verfahrens deutlich positiv. Gegebenenfalls kann dabei das bei der Herstellung von Stärken, insbesondere von chemisch modifizierten Stärken anfallende Abwasser einer Vorbehandlung zur Abtrennung suspendierter Feststoffe unterzogen werden, bevor es dem erfindungsgemäß vorgesehenen Membrantrennverfahren zugeführt wird.

Vorzugsweise wird im Membrantrennverfahren eine Umkehrosmose vollzogen, wobei der erste Volumenstrom nicht nur die im Vergleich zum zugeführten Abwasser höhere Konzentration an gelösten Salzen enthält, sondern auch die organischen Verbindungen des zugeführten Abwassers. Der zweite Volumenstrom stellt in diesem Fall von gelösten Salzen und organischen Verbindungen im Wesentlichen befreites Wasser dar, das im Herstellungsverfahren der Stärke oder modifizierten Stärke wiederverwendet werden kann und eine deutliche Verringerung des zu behandelnden Volumens des ersten Volumenstromes ermöglicht. Somit kann der erste Volumenstrom in wirtschaftlich vertretbarer Weise direkt der thermischen Behandlung zur Rückgewinnung der Salze zugeführt werden.

Alternativ kann im Membrantrennverfahren auch eine Nanofiltration vollzogen werden, wobei der zweite Volumenstrom eine im Vergleich zum zugeführten Abwasser verringerte Konzentration an gelösten Salzen enthält, sowie einen weiteren Anteil an organischen Verbindungen. Diese Ausführung kann beispielsweise dann vorteilhaft sein, wenn ein Teil der organischen Verbindungen in einer Biogasanlage verwertet werden soll, um beispielsweise für die thermische Behandlung zur Abtrennung und Rückgewinnung der gelösten Salze erforderliche Energie zu gewinnen und das Gesamtverfahren somit wirtschaftlicher zu gestalten.

Gemäß einer vorteilhaften Ausführung erfolgt die thermische Behandlung des ersten Volumenstroms in einer Kristallisationsanlage unter Kristallisation gelöster Salze.

Erfindungsgemäß wird der dritte Volumenstrom oder ein von ihm abgeleiteter Volumenstrom einer selektiven Auftrennung zur Abtrennung und Rückgewinnung von 1,2-Propandiol oder Ethylenglykol unterzogen. Die rückgewonnenen organischen Verbindungen 1,2-Propandiol oder Ethylenglykol stellen einen wirtschaftlich verwertbaren Rohstoff dar. Vorzugsweise handelt es sich bei der selektiven Auftrennung zur Abtrennung und Rückgewinnung der organischen Verbindungen um eine Destillation oder Extraktion. Alternativ wäre aber auch der Einsatz von Rektifikation, Adsorption oder Absorption zur selektiven Abtrennung der organischen Verbindungen denkbar.

Dabei wird vorzugsweise vorgeschlagen, dass der dritte Volumenstrom vor der selektiven Auftrennung zur Abtrennung und Rückgewinnung von 1,2-Propandiol oder Ethylenglykol verflüssigt und einer Umkehrosmose zur Abtrennung von Wasser unterzogen wird. Auf diese Weise kann der der selektiven Auftrennung zugeführte Volumenstrom reduziert werden. Die selektive Auftrennung kann damit kleiner und kostengünstiger dimensioniert und der Energieverbrauch reduziert werden.

Um die Mengen sowohl der rückgewonnenen Salze als auch jene der organischen Verbindungen zu erhöhen wird ferner vorgeschlagen, dass die Mutterlauge der Kristallisationsanlage eingedampft und die aus der Eindampfung der Mutterlauge entstehende Verdampferbrüde der selektiven Auftrennung zur Abtrennung und Rückgewinnung der in der Mutterlauge enthaltenen organischen Verbindungen 1,2-Propandiol oder Ethylenglykol zugeführt wird. Die trockenen Rückstände der Mutterlauge werden durch die nun rückgewonnenen und wiederverwendbaren Salze gebildet, und in der Verdampferbrüde sind verwertbare flüchtige organische Verbindungen enthalten, die durch die selektive Auftrennung rückgewonnen werden.

Im Falle der Durchführung einer Nanofiltration als erfindungsgemäßes Membrantrennverfahren kann der zweite Volumenstrom einer Umkehrosmose unterzogen werden, und das Retentat der Umkehrosmose der selektiven Auftrennung zur Abtrennung und Rückgewinnung der im Retentat enthaltenen organischen Verbindungen 1,2-Propandiol oder Ethylenglykol zugeführt werden. Zur Abtrennung der im zweiten Volumenstrom enthaltenen nicht-flüchtigen Komponenten ist es vorteilhaft das Retentat vor der Zuführung zur selektiven Auftrennung einzudampfen, wobei die aus der Eindampfung des Retentats entstandene Verdampferbrüde ebenfalls der selektiven Auftrennung zur Abtrennung und Rückgewinnung der organischen Verbindungen zugeführt wird. Die Eindampfung der Mutterlauge der Kristallisationsanlage und des Retentats können etwa gemeinsam in einem Dünnschichtverdampfer erfolgen.

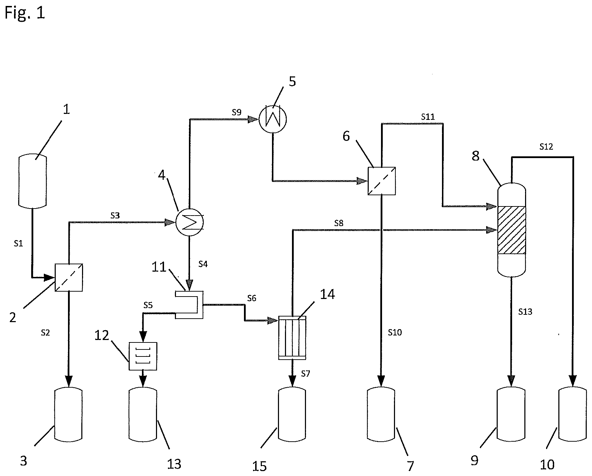

Die Erfindung wird in weiterer Folge anhand eines Ausführungsbeispiels mithilfe der beiliegenden Figuren näher erläutert. Es zeigt hierbei die
Fig. 1 eine schematische Darstellung einer möglichen Ausführungsform des erfindungsgemäßen Verfahrens.

Wie in der Fig. 1 ersichtlich ist, wird das nach Abtrennung und Auswaschung der modifizierten Stärke verbleibende Abwasser S1 zunächst in einem Abwassertank 1 bereitgehalten. Das Abwasser S1 enthält hohe Konzentrationen an organischen Verbindungen und gelösten Salzen. Bei der Herstellung von Hydroxypropylstärke (HPS) beispielsweise fällt Abwasser S1 mit hohen Konzentrationen an 1,2-Propandiol und Natriumsulfat oder auch andere Salze wie Ammoniumsulfat an. Zur Abtrennung suspendierter Feststoffe kann das Abwasser S1 gegebenenfalls einer beispielsweise als Filtrationsstufe ausgeführten Vorreinigung (in der Fig. 1 nicht dargestellt) zugeführt werden, bevor es einer ersten Membranstufe 2 zugeführt wird, in der ein Membrantrennverfahren vollzogen wird, im gezeigten Ausführungsbeispiel der Fig. 1 in Form einer Umkehrosmose.

In der ersten Membranstufe 2 erfolgt eine Trennung des der ersten Membranstufe 2 zugeführten Abwassers. S1 in einen ersten Volumenstrom S3 mit im Vergleich zum zugeführten Abwasser S1 höherer Konzentration an gelösten Salzen und in einen zweiten, von gelösten Salzen im Wesentlichen befreiten Volumenstrom S2. Die organischen Verbindungen befinden sich bei Anwendung einer Umkehrosmose ebenfalls im ersten Volumenstrom S3. Der zweite Volumenstrom S2 ist somit von gelösten Salzen und organischen Verbindungen im Wesentlichen befreites Wasser, das in einem ersten Wassertank 3 gesammelt und im Herstellungsverfahren der Stärke oder modifizierten Stärke, oder auch an anderer Stelle des Betriebes wiederverwendet werden kann. Der erste Volumenstrom S3 weist somit aufgrund der Abtrennung des zweiten Volumenstroms S2 ein deutlich verringertes Volumen gegenüber dem der ersten Membranstufe 2 zugeleiteten Abwasser S1 auf. Dadurch kann der erste Volumenstrom S3 in wirtschaftlich vertretbarer Weise direkt einer Kristallisationsanlage 4 zur thermischen Behandlung des ersten Volumenstroms S3 und Rückgewinnung der gelösten Salze zugeführt werden.

In der Kristallisationsanlage 4 erfolgt eine Eindampfung des ersten Volumenstroms S3 unter Kristallisation der gelösten Salze. Die in der Kristallisationsanlage 4 entstehende Brüde enthält kaum mehr gelöste Salze, aber eine hohe Konzentration an flüchtigen organischen Verbindungen und stellt einen dritten Volumenstrom S9 dar, der zunächst in einem Kondensator 5 verflüssigt wird. Die abzuführende Wärme kann durch eine Wärmerückgewinnung für die thermische Behandlung oder für die nachfolgende Destillation genutzt werden (in der Fig. 1 nicht dargestellt). Der verflüssigte dritte Volumenstrom S9 wird in weiterer Folge gemäß dem Ausführungsbeispiel der Fig. 1 einer zweiten Membranstufe 6 zugeführt, in der er einer Umkehrosmose unterzogen wird. In der zweiten Membranstufe 6 wird eine erste gereinigte Wasserfraktion S10 gewonnen, die in einem zweiten Wassertank 7 gesammelt und im Herstellungsverfahren der Stärke oder modifizierten Stärke, oder auch an anderer Stelle des Betriebes wiederverwendet werden kann.

Aus der zweiten Membranstufe 6 kann des Weiteren eine an organischen Verbindungen hochkonzentrierte Fraktion S11 abgeleitet werden, die in weiterer Folge einer selektiven Auftrennung zur Abtrennung und Rückgewinnung der organischen Verbindungen unterzogen wird. Im gezeigten Ausführungsbeispiel der Fig. 1 wird die selektive Auftrennung in einer Destillationsanlage 8 vollzogen, wobei selektiv die gewünschten organischen Verbindungen als Organikfraktion S13 gewonnen und in einem Sammeltank 9 gesammelt werden können. Bei der Herstellung von Hydroxypropylstärke (HPS) beispielsweise fällt Abwasser S1 mit einer hohen Konzentration an 1,2-Propandiol an, das mittels des Verfahrens der Fig. 1 in der Destillationsanlage 8 als Organikfraktion S13 gewonnen, im Sammeltank 9 gelagert und in weiterer Folge wirtschaftlich verwertet werden kann. Die Organikfraktion S13 wird vorzugsweise gekühlt, wobei die abzuführende Wärme beispielsweise zum Aufheizen der Destillationsanlage 8 oder zur thermischen Behandlung von zugeführten Medien verwendet werden kann. Ein finaler Volumenstrom S12 der Destillationsanlage 8 stellt eine zweite gereinigte Wasserfraktion S12 dar, die in einem dritten Wassertank 10 gesammelt und im Herstellungsverfahren der Stärke und modifizierten Stärke wiederverwendet werden kann. Auch die gereinigte Wasserfraktion S12 wird vorzugsweise gekühlt, wobei die Abwärme im Verfahren zum Aufheizen von Medien genutzt werden kann.

Der Rückstand S4 der Kristallisationsanlage 4 wird zunächst einer Zentrifuge 11 zugeführt, in der die auskristallisierten Salze als erste Salzfraktion S5 abgetrennt und in einem Trockner 12 getrocknet werden. Das getrocknete Salz wird in einem ersten Salzsilo 13 gesammelt. Bei der Herstellung von Hydroxypropylstärke (HPS) beispielsweise fällt Abwasser S1 mit einer hohen Konzentration von Natriumsulfat an, das im ersten Salzsilo 13 gesammelt und im Herstellungsverfahren der modifizierten Stärke wiederverwendet werden kann.

Die die Zentrifuge 11 und die Kristallisationsanlage 4 verlassende Mutterlauge S6 enthält eine hohe Konzentration an Salzen, nicht-verdampfbare Rückstände sowie flüchtige organische Verbindungen. Sie wird einem Dünnschichtverdampfer 14 zugeführt, um die enthaltenen Salze als eine zweite Salzfraktion S7 zu gewinnen, die in einem zweiten Salzsilo 15 gesammelt werden kann. Die Verdampferbrüde S8 des Dünnschichtverdampfers 14 enthält Wasser und flüchtige organische Verbindungen und wird der Destillationsanlage 8 zugeführt, um eine nahezu vollständige Rückgewinnung dieser organischen Verbindungen zu erreichen.

Tatsächlich erweist sich das erfindungsgemäße Verfahren alleine durch Rückgewinnung und Wiederverwertung der rückgewonnenen Salze trotz des Energieaufwands für die thermische Behandlung in der Kristallisationsanlage 4 als wirtschaftlich. Durch die zusätzliche Rückgewinnung und Wiederverwertung der organischen Verbindungen gestaltet sich die wirtschaftliche Bilanz des erfindungsgemäßen Verfahrens deutlich positiv. Zudem können durch die Rückgewinnung von Wasser und gelösten Salzen, die als rückgewonnene Rohstoffe im Herstellungsverfahren der modifizierten Stärke wiederverwendet werden können, die Rohstoffkosten für das Herstellungsverfahren reduziert und der beträchtliche Trinkwasserverbrauch und die abzuleitenden Abwassermengen in der Herstellung von chemisch modifizierten Stärken nahezu vollständig eliminiert werden. Bei der Neuplanung einer Anlage zur Herstellung modifizierter Stärken kann auf eine biologische Abwasserreinigung verzichtet und somit die Entsorgung der darin anfallenden Biomasse (Klärschlamm) vermieden werden. Bei bestehenden Anlagen können mithilfe des erfindungsgemäßen Verfahrens die hydraulische und organische Belastung der biologischen Abwasserreinigungsanlage reduziert und somit Abwasserbehandlungsreserven geschaffen werden.

## Patentansprüche

1. Verfahren zur Aufbereitung von bei der Herstellung von Stärken, insbesondere von chemisch modifizierten Stärken anfallendem und gelöste Salze und organische Verbindungen enthaltendem Abwasser (S1), **dadurch gekennzeichnet, dass** das Abwasser (S1) oder ein die gelösten Salze und die organischen Verbindungen des Abwassers (S1) im Wesentlichen enthaltendes, vorbehandeltes Abwasser (S1) einem Membrantrennverfahren unterzogen wird, in der eine Trennung des dem Membrantrennverfahren zugeführten Abwassers (S1) in einen ersten Volumenstrom (S3) mit im Vergleich zum zugeführten Abwasser (S1) höherer Konzentration an gelösten Salzen und in einen zweiten Volumenstrom (S2) mit im Vergleich zum zugeführten Abwasser (S1) verringerter Konzentration an gelösten Salzen erfolgt, wobei der erste Volumenstrom (S3) einer thermischen Behandlung zur Abtrennung der gelösten Salze und eines dritten, einen Anteil der organischen Verbindungen des Abwassers (S1) enthaltenden Volumenstroms (S9) unterzogen wird, und der dritte Volumenstrom (S9) oder ein von ihm abgeleiteter Volumenstrom einer selektiven Auftrennung zur Abtrennung und Rückgewinnung von 1,2-Propandiol oder Ethylenglykol unterzogen wird.

2. Verfahren nach Anspruch 1, **dadurch gekennzeichnet, dass** im Membrantrennverfahren eine Umkehrosmose vollzogen wird, wobei der zweite Volumenstrom (S2) von gelösten Salzen und organischen Verbindungen im Wesentlichen befreites Wasser ist.

3. Verfahren nach Anspruch 1, **dadurch gekennzeichnet, dass** im Membrantrennverfahren eine Nanofiltration vollzogen wird, wobei der zweite Volumenstrom (S2) einen einen weiteren Anteil der organischen Verbindungen des Abwassers (S1) enthaltenden Volumenstrom darstellt.

4. Verfahren nach einem der Ansprüche 1 bis 3, **dadurch gekennzeichnet, dass** die thermische Behandlung des ersten Volumenstroms (S3) in einer Kristallisationsanlage (4) unter Kristallisation gelöster Salze erfolgt.

5. Verfahren nach einem der Ansprüche 1 bis 4, **dadurch gekennzeichnet, dass** es sich bei der selektiven Auftrennung um eine Destillation handelt.

6. Verfahren nach einem der Ansprüche 1 bis 4, **dadurch gekennzeichnet, dass** es sich bei der selektiven Auftrennung um eine Extraktion handelt.

7. Verfahren nach einem der Ansprüche 1 bis 6, **dadurch gekennzeichnet, dass** der dritte Volumenstrom (S9) vor der selektiven Auftrennung zur Abtrennung und Rückgewinnung von 1,2-Propandiol oder Ethylenglykol verflüssigt und einer Umkehrosmose zur Abtrennung von Wasser unterzogen wird.

8. Verfahren nach Anspruch 4 und einem der Ansprüche 5 bis 7, **dadurch gekennzeichnet, dass** die Mutterlauge (S6) der Kristallisationsanlage (4) eingedampft und die aus der Eindampfung der Mutterlauge (S6) entstandene Verdampferbrüde (S8) der selektiven Auftrennung zur Abtrennung und Rückgewinnung der in der Mutterlauge (S6) enthaltenen organischen Verbindungen 1,2-Propandiol oder Ethylenglykol zugeführt wird.

9. Verfahren nach Anspruch 3 und einem der Ansprüche 4 bis 8, **dadurch gekennzeichnet, dass** der zweite Volumenstrom (S2) einer Umkehrosmose unterzogen wird, und das Retentat der Umkehrosmose der selektiven Auftrennung zur Abtrennung und Rückgewinnung der im Retentat enthaltenen organischen Verbindungen 1,2-Propandiol oder Ethylenglykol zugeführt wird.

## Claims

1. Process for the treatment of wastewater (S1) obtained in the production of starches, in particular chemically modified starches, and containing dissolved salts and organic compounds, **characterized in that** the wastewater (S1) or a pretreated wastewater (S1) substantially containing the dissolved salts and the organic compounds of the wastewater (S1) is subjected to a membrane separation process, in which a separation of the wastewater (S1) supplied to the membrane separation process into a first volume flow (S3) with a higher concentration of dissolved salts in comparison with the supplied wastewater (S1) and into a second volume flow (S2) with a lower concentration of dissolved salts in comparison with the supplied wastewater (S1) is carried out, wherein the first volume flow (S3) is subjected to a thermal treatment for separating the dissolved salts and a third volume flow (S9) containing a fraction of the organic compounds of the wastewater (S1), and the third volume flow (S9) or a volume flow derived therefrom is subjected to a selective fractionation for the separation and recovery of 1,2-propanediol or ethylene glycol.

2. Process according to claim 1, **characterized in that** a reverse osmosis is carried out in the membrane separation process, wherein the second volume flow (S2) is water substantially freed from dissolved salts and organic compounds.

3. Process according to claim 1, **characterized in that** a nanofiltration is carried out in the membrane separation process, wherein the second volume flow (S2) represents a volume flow containing a further fraction of the organic compounds of the wastewater (S1).

4. Process according to one of the claims 1 to 3, **characterized in that** the thermal treatment of the first volume flow (S3) is carried out in a crystallization plant (4) with crystallization of dissolved salts.

5. Process according to claim one of the claims 1 to 4, **characterized in that** the selective fractionation is a distillation.

6. Process according to one of the claims 1 to 4, **characterized in that** the selective fractionation is an extraction.

7. Process according to one of the claims 1 to 6, **characterized in that** the third volume flow (S9), before the selective fractionation for the separation and recovery of 1,2-propanediol or ethylene glycol, is liquefied and subjected to reverse osmosis for the separation of water.

8. Process according to claim 4 and one of the claims 5 to 7, **characterized in that** the mother liquor (S6) of the crystallization plant (4) is evaporated and the evaporator vapor (S8) resulting from the evaporation of the mother liquor (S6) is supplied to the selective fractionation for the separation and recovery of the organic compounds 1,2-propanediol or ethylene glycol contained in the mother liquor (S6).

9. Process according to claim 3 and one of the claims 4 to 8, **characterized in that** the second volume flow (S2) is subjected to reverse osmosis, and the retentate of the reverse osmosis is supplied to the selective fractionation for separating and recovering the organic compounds 1,2-propanediol or ethylene glycol contained in the retentate.

## Revendications

1. Procédé de traitement d'eau résiduaire (S1) produite lors de la fabrication d'amidons, en particulier d'amidons chimiquement modifiés, et contenant des sels dissous et des composés organiques, **caractérisé en ce que** l'eau résiduaire (S1) ou une eau résiduaire prétraitée (S1) contenant sensiblement les sels dissous et les composés organiques de l'eau résiduaire (S1) sont soumises à un procédé de séparation par membrane, dans lequel l'eau résiduaire (S1) amenée au procédé de séparation par membrane est séparée en un premier flux volumique (S3) avec une concentration plus élevée en sels dissous par rapport à l'eau résiduaire amenée (S1) et en un deuxième flux volumique (S2) avec une concentration plus faible en sels dissous par rapport à l'eau résiduaire amenée (S1), dans lequel le premier flux volumique (S3) est soumis à un traitement thermique pour séparer les sels dissous et un troisième flux volumique (S9) contenant une partie des composés organiques de l'eau résiduaire (S1), et le troisième flux volumique (S9) ou un flux volumique dérivé de celui-ci est soumis à une séparation sélective pour séparer et récupérer le 1,2-propanediol ou l'éthylène glycol.

2. Procédé selon la revendication 1, **caractérisé en ce qu'**une osmose inverse est réalisée dans le procédé de séparation par membrane, le deuxième flux volumique (S2) étant de l'eau sensiblement débarrassée des sels dissous et des composés organiques.

3. Procédé selon la revendication 1, **caractérisé en ce qu'**une nanofiltration est réalisée dans le processus de séparation par membrane, le deuxième flux volumique (S2) représentant un flux volumique contenant une autre partie des composés organiques de l'eau résiduaire (S1).

4. Procédé selon l'une des revendications 1 à 3, **caractérisé en ce que** le traitement thermique du premier flux volumique (S3) est effectué dans une installation de cristallisation (4) avec cristallisation de sels dissous.

5. Procédé selon l'une des revendications 1 à 4, **caractérisé en ce que** la séparation sélective est une distillation.

6. Procédé selon l'une des revendications 1 à 4, **caractérisé en ce que** la séparation sélective est une extraction.

7. Procédé selon l'une des revendications 1 à 6, **caractérisé en ce que** le troisième flux volumique (S9) est liquéfié et soumis à une osmose inverse pour séparer l'eau avant la séparation sélective pour séparer et récupérer le 1,2-propanediol ou l'éthylène glycol.

8. Procédé selon la revendication 4 et l'une des revendications 5 à 7, **caractérisé en ce que** l'eau mère (S6) de l'installation de cristallisation (4) est évaporée et la vapeur d'évaporation (S8) résultant de l'évaporation de l'eau mère (S6) est amenée à la séparation sélective pour séparer et récupérer les composés organiques 1,2-propanediol ou éthylène glycol contenus dans l'eau mère (S6).

9. Procédé selon la revendication 3 et l'une des revendications 4 à 8, **caractérisé en ce que** le deuxième flux volumique (S2) est soumis à une osmose inverse, et le rétentat de l'osmose inverse est amené à la séparation sélective pour séparer et récupérer les composés organiques 1,2-propanediol ou éthylène glycol contenus dans le rétentat.
